# EUROPEAN PATENT SPECIFICATION

(11) **EP 3 314 305 B1**
(45) Date of publication and mention of the grant of the patent: **10.06.2020**
(21) Application number: 16814812.0
(22) Date of filing: 22.06.2016
(51) Int. Cl.: G01T 1/178, G01N 1/22, G01N 33/00, G01T 7/02

(54) **RADON DETECTOR COMPRISING MULTIPLE DETECTOR LOCATION AREAS**
RADONDETEKTOR MIT MEHREREN DETEKTORLOKALISIERUNGSZONEN
DÉTECTEUR DE RADON À MULTIPLES ZONES D'EMPLACEMENT DE DÉTECTEUR

(30) Priority: 26.06.2015 SE 1550891
(43) Date of publication of application: 02.05.2018
(73) Proprietor: Radonova Laboratories AB, 751 38 Uppsala (SE)
(72) Inventor: BYSTRÖM, Kalle, 755 92 Uppsala (SE); RÖNNQVIST, Tryggve, 756 45 Uppsala (SE)
(74) Representative: Aros Patent AB
(86) International application number: PCT/SE2016/050602
(87) International publication number: WO 2016/209150

(56) References cited:
- WO-A1-2008/080753
- WO-A1-2008/080753
- US-A- 4 704 537
- US-A- 4 980 550
- US-A- 5 068 538
- US-A- 5 132 535
- US-A1- 2003 030 444
- US-B1- 8 153 959

## Description

### TECHNICAL FIELD

The proposed technology generally relates to detection of radon gas and in particular to a radon detector and a method for measuring radon content.

### BACKGROUND

Radon is a radioactive gas that occurs naturally in the ground in many places. Radon is also present in some types of building materials, e.g. Autoclaved aerated concrete (AAC) comprising uranium. The radon gas may be released from the ground or the buildings and may penetrate into buildings and apartments. High concentrations of radon may also appear in deep wells. Radon may cause lung cancer when it is inhaled. Thousands of people are struck by cancer each year due to exposure for radon gas.

The radon concentration in the indoor air varies with the time of the year, mainly because of differences in temperature and wind conditions. The concentration also typically varies during the day, from one room to another, and depending on the efficiency of the ventilation system. In places where radon is expected to be present, due to ground conditions or construction material, the radon concentration is of interest to measure in order to allow countermeasures.

A common method for measurement of radon content in air utilizes nuclear track detectors, e.g. nuclear track films. A film is mounted in a closed detector compartment and any alpha decay from the radon gas or radon decay products is registered in the film. The detector compartment is however designed for allowing radon gas to diffuse in and out from the detector compartment. The detector is left in the room in which the measurements are to be performed for a certain time, typically at least a couple of days and usually up to two or three months, and the nuclear track detector is then analyzed to determine the radon gas content.

In the US patent US 5,068,538, a radon monitor with sealable detecting chamber is disclosed. A compact electrically conductive housing includes a plurality of access ports defined in the top section of the housing to permit radon gas (²²²Rn) to diffuse into the housing. The access ports are covered by a diffusion barrier such as a layer of foam rubber sheet inside the housing. The bottom section of the housing includes an integrally molded, electrically conductive pedestal having three recessed wells therein. Two wells contain a stacked pair of an SSNTD on top of a calcium fluoride TLD. The third well contains the same components, but with the TLD on top of the SSNTD, and TLD of this stacked pair is pre-dosed with gamma-rays. The pedestal is covered with a film of electrically neutral aluminized MYLAR (polyester film). A background protection disc prevents substantial exposure of the SSNTDs to radon radiation until the desired measurement period beings, once the monitor reaches the intended measurement site. The disc further prevents additional exposure once the intended measurement period is concluded.

As mentioned above, the radon concentration may vary considerably from time to time and also depending on the activities and/or ventilation that is present in the surroundings. For measurements in e.g. factories or offices that are empty during a considerable time and where e.g. the ventilation follows the intensity of activities, completely different radon gas concentrations may be present during different times. Such fast variations in radon concentrations are not possible to record by prior-art passive radon detector systems.

### SUMMARY

It is an object to provide means and methods for enabling measurements of short-term radon gas content variations.

This and other objects are met by embodiments of the proposed technology.

According to a first aspect, there is provided a radon detector as defined in independent claim 1, comprising a base portion, a cover portion and a shield arrangement. The cover portion is arranged for being removably attached to the base portion. The cover portion, when being attached to the base portion, houses a contained gas volume between the cover portion and the base portion. The cover portion and the base portion, when being attached to each other, allow diffusion of gas between a surrounding into the contained gas volume. The base portion has at least two detector location areas, enabling mechanical arranging of nuclear track detector means to the base portion. The shield arrangement comprises at least one shield and a shield actuator. The shield actuator is arranged for mechanically moving a shield of the at least one shield between a closed position and an open position, for each of the detector location areas, so that a closing of one detector location area is connected to an opening of another detector location area. Thereby, the shield of the at least one shield in the closed position of a respective the detector location areas prevents a line of sight between at least a part of the contained gas volume and the respective said detector location area. Furthermore, the shield of the at least one shield in the open position of the particular detector position allows a line of sight between the at least a part of the contained gas volume and the respective detector location area. The shield actuator is controllable from outside the contained gas volume.

A second aspect of the embodiments relates to a method for measuring radon content as defined in independent claim 11, which comprises mounting of nuclear track detector means in at least two detector location areas in a radon detector having an contained gas volume in diffusion contact with a surrounding. The radon detector is prepared to enable, when the radon detector is placed at a measurement location, mechanically moving of a shield between a closed position and an open position, for each of the detector location areas, so that a closing of one detector location area is connected to an opening of another detector location area. The shield in the closed position of a respective detector location areas prevents a line of sight between at least a part of the contained gas volume and the respective detector location area. The shield of the at least one shield in the open position of the particular detector position allows a line of sight between the at least a part of the contained gas volume and the respective detector location area. A response of the nuclear track detector means are analyzing for presence of radon.

An advantage of the proposed technology is that it enables to switch between nuclear track detector means of different detector location areas, thereby enabling a time-selective detection. Other advantages will be appreciated when reading the detailed description.

### BRIEF DESCRIPTION OF THE DRAWINGS

The embodiments, together with further objects and advantages thereof, may best be understood by making reference to the following description taken together with the accompanying drawings, in which:
FIGS. 1A-B are schematic illustrations of radon detection in open and closed configurations;
FIG. 2 is a schematic illustration of an embodiment of a base portion of a radon detector;
FIG. 3 is a schematic illustration of an embodiment of a base portion and a shield arrangement of a radon detector;
FIG. 4 is a schematic illustration of an embodiment of a radon detector;
FIG. 5 is a schematic cross-sectional view of the embodiment of Fig. 4;
FIG. 6 is a schematic illustration of an embodiment of a base portion, a shield arrangement and a time stamp arrangement of a radon detector;
FIG. 7 is a schematic illustration of another embodiment of a radon detector;
FIG. 8 is a schematic illustration of another embodiment of a base portion of a radon detector;
FIG. 9 is a schematic illustration of yet another embodiment of a base portion of a radon detector;
FIG. 10 is a schematic illustration of yet another embodiment of a radon detector;
FIG. 11 is a schematic illustration of yet another embodiment of a radon detector;
FIG. 12 is a schematic illustration of another embodiment of a base portion and a shield arrangement of a radon detector;
FIG. 13 is a schematic illustration of yet another embodiment of a base portion and a shield arrangement of a radon detector;
FIG. 14 is a schematic illustration of yet another embodiment of a base portion and a shield arrangement of a radon detector; and
FIG. 15 is a flow diagram of steps of an embodiment of a method for measuring radon content.

### DETAILED DESCRIPTION

Throughout the drawings, the same reference designations are used for similar or corresponding elements.

Radon gas is radio-active and is e.g. comprised in the decay chain of uranium. The most common isotope ²²²Rn has a half-life of 3.8 days and decays with alpha decay. An alpha decay means that the original radon nucleus decays by sending out a helium nucleus, i.e. an alpha particle. The daughter nuclide of the Rn decay is ²¹⁸Po, which also is radio-active. ²¹⁸Po also has a dominating alpha decay with a half-life of 3 minutes. Also some other of the decay products are radioactive and may also undergo a further decay process, emitting further alpha particles. These alpha particles are emitted with a velocity and travel until they reach any matter. Alpha particles are easily stopped, even a paper will prohibit a vast majority of impinging alpha particles to continue their linear path.

Nuclear track detector means are used for detecting alpha particles. The nuclear track detector means do not record any gamma rays. The operation, as such, is well known by a person skilled in the art and the details in the operation of such devices will therefore not be further discussed. A typical, non-limiting, material for use as a passive nuclear track detector means is a polymer material denoted CR39. Also other similar material, known in prior art as passive nuclear track detector means, can be used. Due to the easiness of stopping alpha radiation, a nuclear track detector means will only be able to detect alpha radiation originating at a position within line-of-sight to the nuclear track detector means. Furthermore, only alpha radiation emitted in the direction towards the nuclear track detector means is possible to detect, since there are no ways for focusing or refracting alpha radiation. By knowing the volume within sight from the nuclear track detector and the relative geometry, the number of detected alpha particles can be associated with a particular radon concentration in the gas. The general principles for such detection and calculations are well known within the art of radon detectors and is therefore well known, as such, by any person skilled in the art.

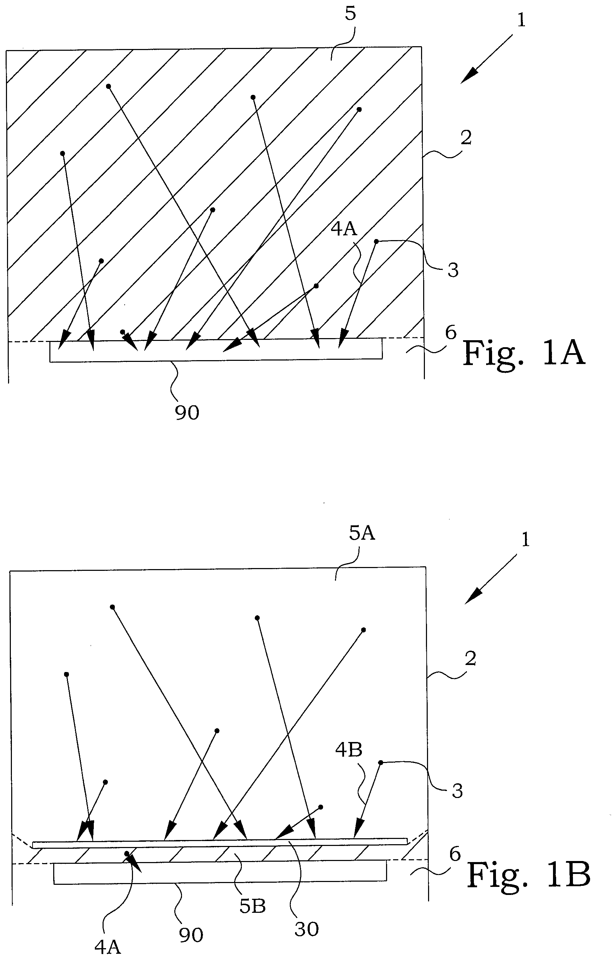

Fig. 1A illustrates schematically an embodiment of a radon detector 1. A nuclear track detector means 90 is configured to detect alpha radiation impinging onto the surface of the nuclear track detector means 90. An enclosure 2 defines a contained gas volume 5. The gas volume 5 may have allow diffusion of gas between a surrounding into the contained gas volume 5, as indicated by the openings 6 at the sides of the nuclear track detector means 90. Radon gas atoms 3 may decay and send out alpha particles 4A. If the path of the alpha particles 4A is directed towards the nuclear track detector means 90, the alpha particles 4A will impinge onto the nuclear track detector means 90 and be detected. Alpha particles emitted in other directions will not reach the nuclear track detector means 90 and are not detected. Such alpha particles are neglected in Fig. 1A. Radon gas existing in the surroundings of the enclosure 2 will not contribute to the detected alpha radiation unless the radon gas diffuses into the contained gas volume 5. The contained gas volume 5, from which alpha radiation can be detected by the nuclear track detector means 90 has been marked in Fig. 1A.

In Fig. 1B, a shield arrangement 30 is provided just in front of the nuclear track detector means 90. A major part 5A of the contained gas volume cannot any longer contribute with detectable alpha radiation, since the alpha radiation 4B will be stopped by the shield arrangement 30. Only the small volume 5B just in front of the nuclear track detector means 90 will still contribute with detectable alpha radiation 4A. By making the ratio between the contained gas volume 5 in Fig. 1A and the limited volume 5B as large as possible, the fraction of detected alpha radiation from the limited volume 5B can be almost neglected. This opens up a possibility to "open" and "close" a nuclear track detector means 90.

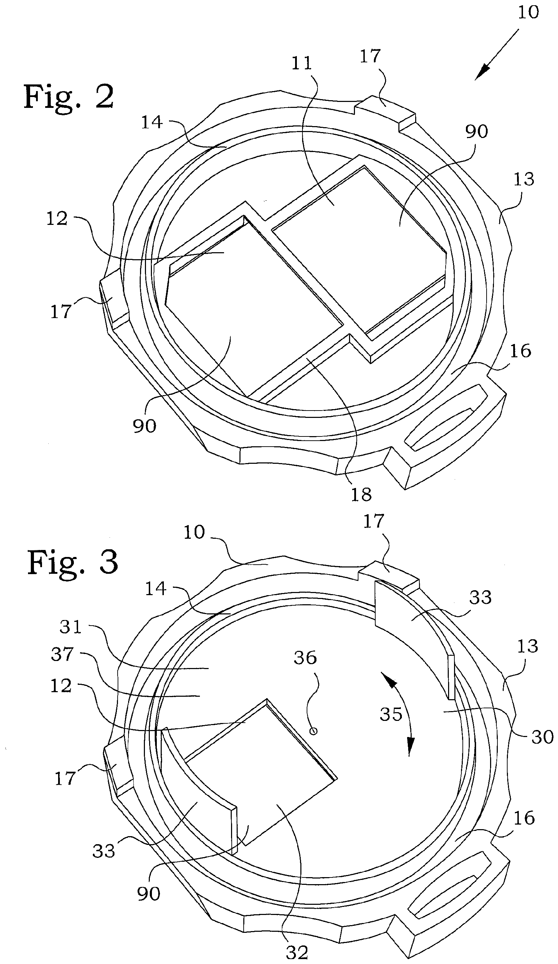

Fig. 2 illustrates schematically an embodiment of a base portion 10 of a radon detector in an elevation view. In this embodiment, the base portion 10 has an outer edge 13 and an inner rim 14, defining a groove 16 between them. Protrusions 18 provided inside the inner rim 14 and protruding from a main plane of the base portion 10 defines a first detector location area 11 and a second detector location area 12. In this particular embodiment, two nuclear track detector means 90 are provided in a respective detector location area 11, 12. The surface of the nuclear track detector means 90 are provided at a plane just below the plane of the upper surface of the protrusions 18. The nuclear track detector means 90 are placed in the respective detector location area 11, 12. In other words, the base portion 10 of the present embodiment has two detector location areas, enabling mechanical arranging of nuclear track detector means 90 to the base portion 10. The outer edge 13 presents two elevated portions 17, which, as will be discussed further below, are to be used as rotation stops.

Fig. 3 illustrates the base portion of Fig. 2, when a shield arrangement 30 has been provided in top of the base portion 10. The shield arrangement 30 comprises a shield 37, in the present embodiment in the shape of a circular disc 31 that fits within the inner rim 14 of the base portion 10. In the present embodiment, the circular disc 31 defines a hole 32 which when positioned above one of the detector location areas, in this example the second detector location area 12 reveals a part of a nuclear track detector means 90 provided in the second detector location area 12. The shield arrangement 30 further comprises engagement tabs 33 for engagement with a cover portion, as will be discussed further below. The circular disc 31 is freely rotatable, as indicated by the double arrow 35, around a central axis 36. By rotating the circular disc 31 180 degrees, the hole 32 will be positioned just above the first detector location area 11, and any nuclear track detector means 90 being present in that position will then be viewable from above.

The protrusions 18 (Fig. 2) defines in the present embodiment a plane. The circular disc 31 is thus supported by the protrusions and the bottom plane of the circular disc 31 is placed very close above the nuclear track detector means 90. This prohibits the nuclear track detector means 90 to leave the respective detector location area 11, 12.

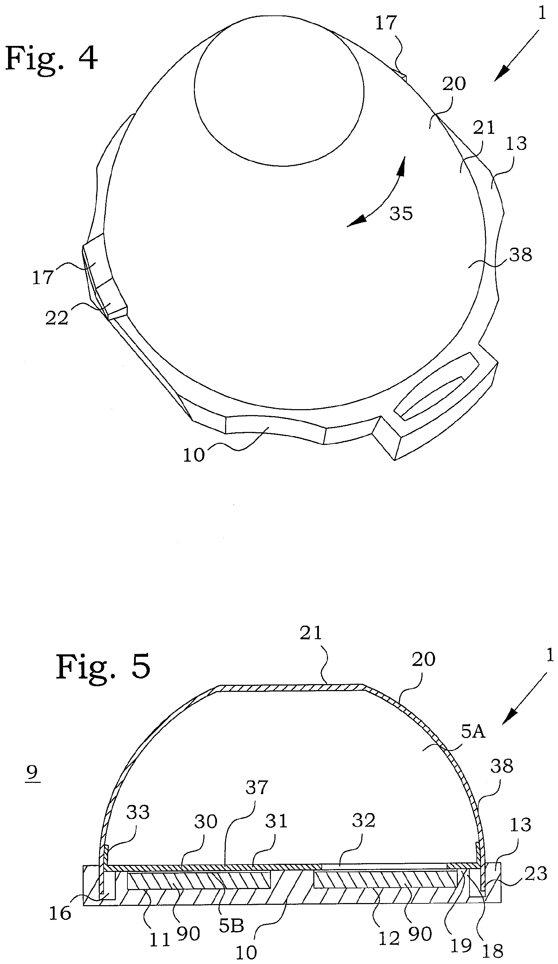

Fig. 4 illustrates an embodiment of a radon detector 1, comprising the base portion 10 of Figs. 2 and 3 and the shield arrangement of Fig. 3. The radon detector 1 further comprises a cover portion 20, arranged for being removably attached to the base portion 10. In the present embodiment, the cover portion 20 has the general shape of a half sphere 21. The cover portion 20 comprises in this embodiment also a stop tab 22, which will be discussed further below.

Fig. 5 illustrates in a simplified schematic manner a cross-sectional view of the radon detector 1 of Fig. 5. Edges 23 of the half sphere 21 are fitted into the grooves 16 of the base portion 20. The cover portion 20, when being attached to the base portion 10, houses a contained gas volume 5A between the cover portion 20 and the base portion 10. There are minor openings or slits between the base portion 10 and the cover portion 20 which allow for a slow exchange of gas between the contained gas volume and the surrounding 9. In a typical example, a difference in radius between the inner wall of the edge 23 and the outer wall of the cover portion may amount to 0.1-0.2 mm, which is enough for ensuring a balance in radon content between the surroundings 9 and the contained gas volume 5A. In other words, the cover portion 20 and the base portion 10, when being attached to each other, allow diffusion of gas between a surrounding 9 into the contained gas volume 5A.

In this embodiment, the cover portion 20 is engaged to the engagement tabs 33 of the shield arrangement 30, and when rotating the cover portion 20, as indicated by the double arrow 35 in Fig. 4, the shield arrangements 30 follows in a rotational displacement around the central axis 36 (Fig. 3). In other words, the shield actuator 38 is integrated in the cover portion 20. In this embodiment, the cover portion 20 therefore also has the function of a shield actuator 38. In other words, the shield arrangement 30 comprises at least one shield 37 and a shield actuator 38. The shield actuator 38 is arranged for mechanically moving the shield 37 of the at least one shield between a closed position and an open position, for each of the detector location areas 11, 12. In the present embodiment, the shield arrangement 30 comprises a single shield 37, common for all detector location areas 11, 12. In the present embodiment, the shield actuator 38 is arranged for mechanically moving the shield 37 between the closed position and the open position coupled for the two different detector location areas 11, 12. Since the cover portion 20 is the outermost part of the radon detector 1, the shield actuator 38 is controllable from outside the contained gas volume 5A.

In the present embodiment, the main parts of the shield arrangement 30 is provided as a separate part, and only the shield actuator 38 is included or integrated in the cover portion 20. However, in an alternative embodiment, the entire shield arrangement 30 may be provided as an integrated part in the cover portion 20.

In the present embodiment, the mechanical movement thus comprises a rotating movement. However, as discussed further below, other types of movements can also be utilized. Furthermore, in the present embodiment, the mechanical movement is parallel to a main inner surface of the base portion 10. However, also here, in other embodiments, movements in other planes can also be feasible.

In the situation depicted in Fig. 5, the second detector location area 12 is located below the hole 32 in the circular disc 31, which means that alpha decay of radon gas in almost the entire contained gas volume 5A may reach the nuclear track detector means 90 arranged in the second detector location area 12. At the contrary, the second detector location area 11 is located below the shield 37, which means that most of the alpha decay of radon gas in the contained gas volume 5A cannot reach the nuclear track detector means 90 arranged in the first detector location area 11. Only alpha decays occurring in the small volume 5B between the shield 37 and the nuclear track detector means 90 arranged in the first detector location area 11 may give rise to alpha radiation detection in the nuclear track detector means 90 arranged in the first detector location area 11. In other words, the shield 37 in the closed position of a respective detector location area 11, 12 prevents a line of sight between at least a part of the contained gas volume 5A and the respective detector location area 11, 12 and wherein the shield in the open position of the particular detector position 11, 12 allows a line of sight between said part of the contained gas volume 5A and the respective detector location area 11, 12. Preferably, said at least a part of the contained gas volume 5A is a main part of the contained gas volume. The difference between the probability that an alpha decay will occur in the volume that is "seen" through the hole 32 of the shield 37 and in the volume 5B will then be large.

The shield 38 in the closed position of a respective detector location area 11, 12 prevents alpha particles from radon decay within said at least a part of the contained gas volume 5A to reach respective detector location areas 11, 12. Likewise, the shield 38 in the open position of the particular detector position allows alpha particles from radon decay within said at least a part of the contained gas volume 5A to reach the respective detector location area 11, 12.

As discussed further above, the protrusions 18 defines in the present embodiment a plane, on which the circular disc 31 moves. The protrusions 18 thereby constitutes a spacer 19, defining a plane above a main inner surface of base portion 10. The shield 37 in a closed position is then positioned juxtaposed to the plane, and preferably in contact with the spacer 19. The spacer 19 facilitates the juxtaposition of the shield 37 and the upper surface of the nuclear track detector means 90. In a typical design, the distance between the upper surface of the nuclear track detector means 90 and the bottom of the shield 37 can be kept as low as 0.05-0.1 mm without hazarding the nuclear track detector means 90. The close relation between the shield 37 and the upper surface of the nuclear track detector means 90 reduces a remaining gas volume 5B between the shield 37 in the closed position and a respective detector location area 11, 12 in presence of a nuclear track detector means 90. Preferably, this remaining gas volume 5B is less than 10% of the contained gas volume 5A, more preferably less than 3% of the contained gas volume 5A, and most preferably less than 1% of said contained gas volume 5A.

As mentioned before, in the present embodiment, the shield actuator 38 is integrated in the cover portion 20. In the present embodiment, the entire shield arrangement 30 is integrated in or attached to the cover portion 20.

By having radon detector that is enabled to separate measurements, information about differences in time can be achieved. If the radon detector e.g. is mounted in an areas in which there is activity during day time and where there is a low activity during the nights, there may be differences also in the radon concentration in the air. Furthermore, if e.g. the ventilation system is time controlled, such differences may be further increased. It might therefore be of interest to measure the radon activity separately for these different time periods.

If the embodiment of Figs 2-5 is used, one can e.g. let the nuclear track detector means positioned at the first detector location area measure the radon content in the contained gas volume during the day and turn the shield actuator in the evening so that the nuclear track detector means positioned at the second detector location area is permitted to measure the radon content in the contained gas volume. In such a way, time-separated measurements are achieved.

The switching between the different measurement modes can be performed manually. Manual operation is always associated with a certain degree of uncertainty. It is not fully guaranteed that the planned switching scheme is followed. It is not fully guaranteed that any notations about switching times are accurate. To this end, a preferred embodiment of the presently described technique also comprise a time stamp arrangement.

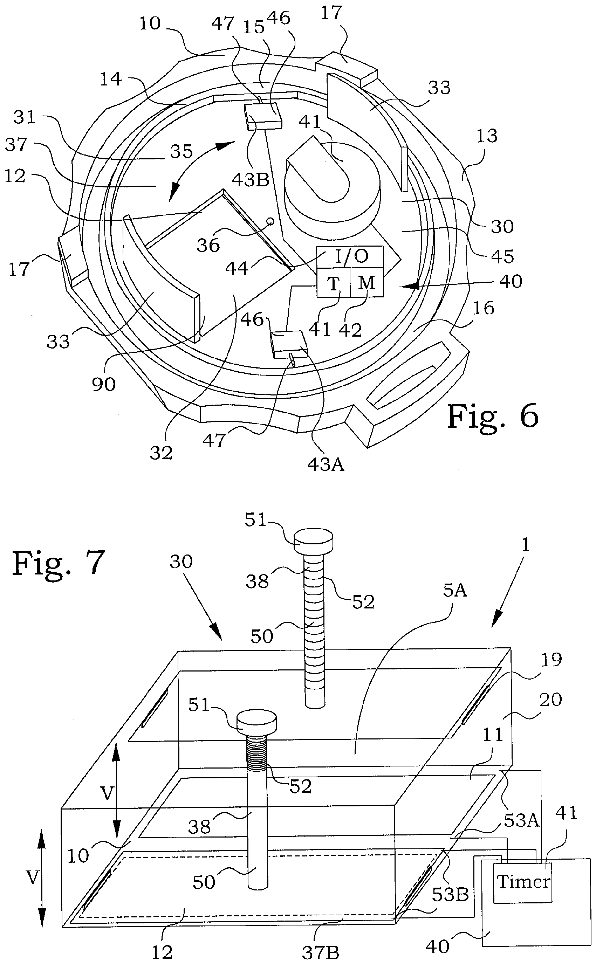

Fig. 6 illustrates another embodiment of parts of a radon detector. The base plate 10 is major parts the same as in earlier embodiments. However, in the present embodiment, the inner rim 14 is provided with a thickened segment 15 at one position. The radon detector of the present embodiment further comprises a time stamp arrangement 40. In this embodiment, the time stamp arrangement 40 is integrated in or attached to the shield arrangement 30. A circuit board 45 is provided, attached to the circular disc 30, on which circuit board 45 components of the time stamp arrangement 40 are arranged. In an alternative embodiment, the circular disc could be constituted by the circuit board 45 itself. The time stamp arrangement 40 comprises a battery for powering the time stamp arrangement 40. The time stamp arrangement 40 further comprises a timer 41, a memory 42 and a memory reading port 44.

The time stamp arrangement 40 further comprises a switch structure 46, in this embodiment comprising a first mechanical switch 43A and a second mechanical switch 43B. A spring loaded sensor pin 47 is provided in a radial direction out from the respective mechanical switch 43A, 43B. In its outermost position, the spring loaded sensor pin 47 reaches close to the main part of the inner surface of the inner rim 14. Such a situation is depicted for the first mechanical switch 43A. However, the second mechanical switch 43B is in the present situation placed at the sector of the thickened segment 15. The tip of the spring loaded sensor pin 47 thereby comes into contact with the thickened segment 15 and is pushed slightly into the housing of the second mechanical switch 43B. In other words, the switch structure 40 is arranged to mechanically interact with a part of the base portion 10 when the shield 37 is in the open position. The second mechanical switch 43B interprets this situation to conclude that the shield arrangement 30 is positioned in an open position relative to the second detector location area 12. In other words, when the spring loaded sensor pin 47 is pushed inwards, there is a line-of-sight between any nuclear track detector means 90 provided at the second detector location area 12 and the contained gas volume. A signal is sent to the timer 41, which records the time of when the switching was performed and stores it in the memory 42. When the shield arrangement 30 is moved, in this embodiment rotated 35, and the contact between the thickened segment 15 and the spring loaded sensor pin 47 of the second mechanical switch 43B is broken, a new signal is sent to the timer 41, which then records the time of when the contact breaking was performed and stores it in the memory 42. In such a situation, the shield arrangement 30 is no longer positioned in an open position with respect to the second detector location area 12. In an alternative embodiment, the actual time instances are not recorded as such, but only the time for when the shield arrangement was placed in the open position with respect to the second detector location area 12.

In other words, the switch structure 46 is arranged to interact with the timer 41 when the shield 37 is mechanically moved from the closed position to the open position and when the shield 37 is mechanically moved from the open position to the closed position, for at least one of the detector location areas 11, 12. The timer 41 is arranged to store information in the memory 42 representing at least an open time for said at least one of the detector location areas. The open time is thus a time difference between the shield being mechanically moved from the closed position to the open position and the shield being mechanically moved from the open position to the closed position.

In a preferred embodiment, the switch structure 46 is an integrated part of the shield arrangement 30.

If the shield 37 in Fig. 6 is rotated half a turn, the opening 32 will be positioned above the first detector location area 11 instead. The nuclear track detector means 90 present at first detector location area 11 will get in direct contact with the contained gas volume. In such a position, the first mechanical switch 43A comes into a location where the spring loaded sensor pin 47 of the first mechanical switch 43A interacts with the thickened segment 15. In analogy with the above described procedure, a signal can be provided to the timer 41 for recording of a time stamp, or the start of a time period measurement. Similarly, the end of such an interaction is signaled and an open time for the first detector location area can be achieved, or alternatively a stop time. The time stamp arrangement 40 is thus arranged to store an open time for each of the detector location areas 11, 12 separately.

As mentioned briefly above, the open time could be just a duration of the shield arrangement being in an open position. Preferably, however, also the individual start and stop times are stored. This enables a tracking of the actual measurement period, not only the length of the measurement period. In other words, the open time comprises a time for when the shields mechanically moved from the closed position to the open position and a time for when the shield is mechanically moved from the open position to the closed position.

By having access to opening and closing times for all detector location areas, one has information about not only the total time for the measurements, but also during e.g. which time of the day of the week the measurements are performed. Furthermore, it can be found if any inaccurate handling of the radon detector has been performed, e.g. if the detector has been in an intermediate position, between the two intended measurement positions, for any significant time. The quality of the measurements can thereby be confirmed.

When a measurement period is over, the nuclear track detector means 90 are analyzed according to prior-art procedures to determine the number of detected alpha particles. The timing information contained in the memory 42 is then also read out from the memory 42 by using the memory reading port 44. This reading port 44 can be configured in many different ways, all known as such in prior art. The reading can e.g. be performed by mechanical connections, by IR communication, by Bluetooth communication, etc. The details of the reading out are not of any particular importance for the present ideas to be achieved and are not further described, since any person skilled in the art has all necessary skills. Once the timing information of the memory 42 is read, this information can be associated with the information from the analysis of the nuclear track detector means 90.

In the embodiments presented above, the mechanical configuration is based on a relative rotational movement, with movements in the main plane of the shield. Those embodiments are also based on a shield arrangement having one shield that is used in common for opening and closing of the different detector location areas. However, numerous of possible alternative configurations are possible. A few of these alternatives are presented below as non-limiting examples of how the general geometries and movements may be varied. However, the person skilled in the art knows that this set of configuration is not a complete set.

In Fig. 7, in a partially transparent schematic illustration, an embodiment of a radon detector 1 has a shield arrangement 30 that comprises one separate shield 37A, 37B for each of the detector location areas 11, 12. A first shield 37A is controlled in a vertical direction V by means of a maneuvering stick 50 with a knob 51. A coil spring 52 is provided around the maneuvering stick 50 tending to push the knob 51 upwards. The first shield 37A is in the illustration positioned in an open position, where the associated first detector location area 11 is in contact with the contained gas volume 5A. The second shield 37B is positioned in a closed position, where the associated second detector location area 11 is hidden from the contained gas volume 5A, as illustrated by the broken lines. The maneuvering stick 50 of the second shield 37B is locked in this position by a (not shown) ratchet. The first and the second shields 37A, 37B are provided with an electrically conducting lower surface. When the shields 37A, 37B are locked in a respective closed position, the lower surface of the shields 37A, 37B comes into contact with two respective electrical connections 53A, 53B, in turn connected to the timer 41. The timer 41 is configured to determine if there is an electrical contact between the electrical connections 53A, 53B and can thereby conclude if the corresponding shield 37A, 37B is positioned in the respective closed position. Thereby, time stamps of any change of position of the shields 37A, 37B can be registered.

In this embodiment, the movements of the shields 37A, 37B are possible to perform independently from each other. In other words, the shield actuator 38 is arranged for mechanically moving the shields 37A, 37B between the closed position and the open position independently for different detector location areas 11, 12. This enables further variations of measurement setups, where the different detector location areas 11, 12 can be used for measurements one at a time, or simultaneously, or where none of the detector location areas 11, 12 are used for measurements. For instance, if alternating measurements of the two detector location areas 11, 12 are performed during a measurement period, both measurements can be stopped when the period is over and the radon detector is transported to an analysis site.

Furthermore, in the embodiment of Fig. 7, the mechanical movement of the shields comprises a linear movement V. This movement is furthermore vertical, i.e. perpendicular to the surface of the detector location areas 11, 12. In alternative embodiments, the movement of the shields may be in any other direction as well, in certain embodiments the mechanical movement is parallel to a main inner surface of the base portion and in other embodiments the mechanical movement has at least a component transverse to a main inner surface of the base portion.

In the embodiment of Fig. 7, a narrow spacer 19 is provided at the edge of a lower surface of each shield 37A, 37B. The thickness of the spacer 19 is slightly larger than the thickness of the nuclear track detector means that are intended to be provided at the detector location areas 11, 12. This ensures that the shields 37A, 37B can be positioned very close to a surface of any nuclear track detector means without risking to damage the nuclear track detector means. Thus in the present embodiment, the shield arrangement 30 comprises the spacer 19.

In the embodiment of Fig. 7, the shield actuator 38 is provided separate from the cover portion 20.

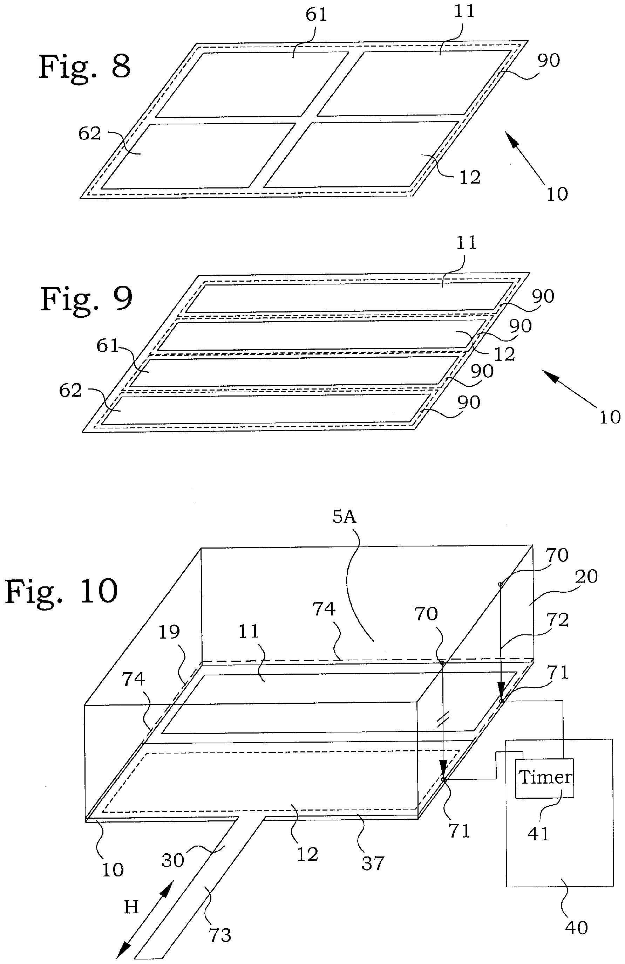

The division of the detector location areas can be altered in many different ways. In Fig. 8, a base portion 10 of another embodiment is illustrated. Here four different detector location areas 61-64 are defined provided in a matrix setup. The use of more than two detector location areas opens up for even more variable measurement setups, where further division of measurement times can be achieved. For instance, one detector location area can be used for measurements daytime during working days, one detector location area can be used for measurements during the night after a working day, one detector location area can be used for measurements during longer inactivity periods, such as weekends or holidays, and one detector location area can be used for control measurements during transportation of the radon detector to and from the site of the measurements. Anyone skilled in the art realizes that the possible variations in use are very large.

In this embodiment, a nuclear track detector means 90 intended to be positioned at the detector location areas 11-12, 61-62 is indicated by broken lines. A single nuclear track detector means 90 is thus placed over the different detector location areas 11-12, 61-62. After measurements are performed, different parts of the nuclear track detector means 90, corresponding to the different detector location areas 11-12, 61-62, are analyzed separately, to distinguish the measurements results from the associated detector location areas 11-12, 61-62.

A common nuclear track detector means 90 for more than one detector location area can be used for any combination of detector location areas 11-12, 61-62. For instance, the embodiments of Figs. 2-6 can be modified to receive a single nuclear track detector means 90, of which different detector areas correspond to the different detector location area.

In Fig. 9, another embodiment of a base portion 10 is illustrated. Here the detector location areas 11-12, 61-62 are provided as rectangular areas provided side by side. Also here, a single nuclear track detector means 90 or separate nuclear track detector means 90, which is indicated by broken lines, can be used for the different detector location areas 11-12, 61-62.

Another embodiment of a radon detector 1 is schematically illustrated in Fig. 10. A linear movement of a single shield 37 is here used to switch measurement between the two detector location areas 11, 12. The shield 37 is moved by pulling and pushing a tab 73 parallel to the detector location areas 11, 12, as indicated by the double arrow H. The tab 73 will partially cover the second detector location area 12 also in the open position. However, if the tab 73 is narrow, this will not substantially influence the accuracy of the measurements.

In this embodiment, a time stamp arrangement 40 is also provided. A small light source 70 provides a narrow light beam 72 directed towards one side of each detector location area 11, 12. A light detector 71 at the base portion 10 detects the light, which indicates that the shield 37 is not present and the corresponding first detector location area 11 is in a measuring mode. The light detector at the side of the second detector location area 12 is, however, screened by the shield 37 and no light is detected. The timer 41 is configured to monitor if the light detectors 71 are detecting any light and can register the times of any change of the status.

The shield 37 runs in a recess 74 in the wall of the cover portion 20. The spacer 19 constituted by the wall section below the recess 74 ensures that the shield is placed close to the detector location areas 11, 12 but without risking to damage any nuclear track detector means provided there.

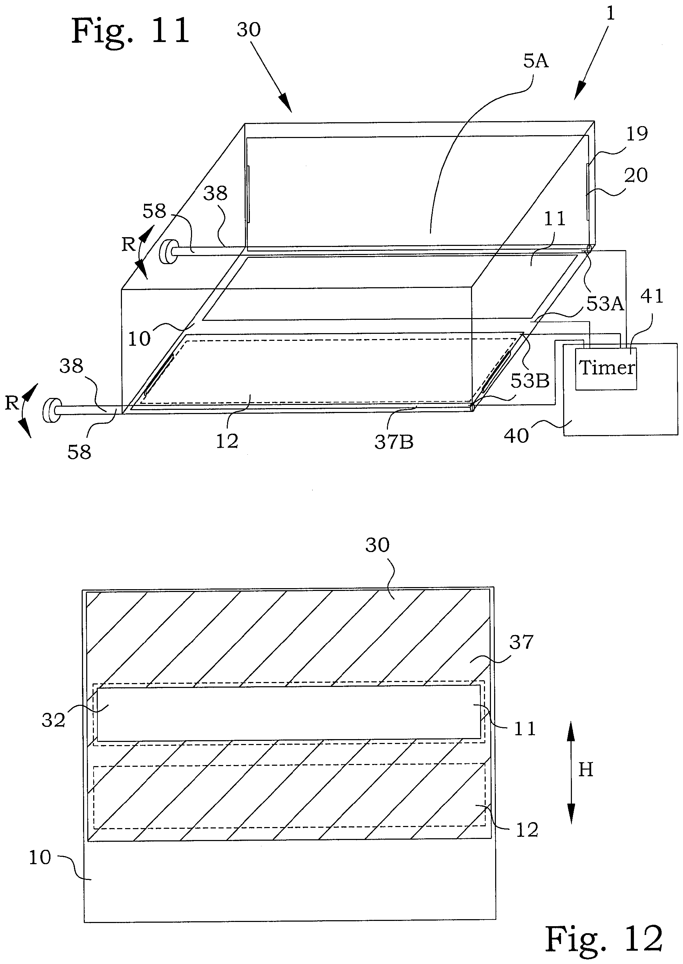

In Fig. 11, an embodiment of a radon detector 1 is illustrated, which have similarities with the embodiment of Fig. 7. However, here a rotational movement of the shields 37 is used to independently open or close the detector location areas 11, 12, by use of turning, indicated by the double arrows R, rods 58 at the side of the radon detector 1.

Fig. 12 illustrates another embodiment of a base portion 10 and a shield arrangement 30, linearly movable. In this embodiment, the closing of one detector location area 11 is connected to the opening of the other detector location area 12. This is achieved by utilizing a common shield 37 with one hole 32, defining the area through which the detection of alpha particles can be performed.

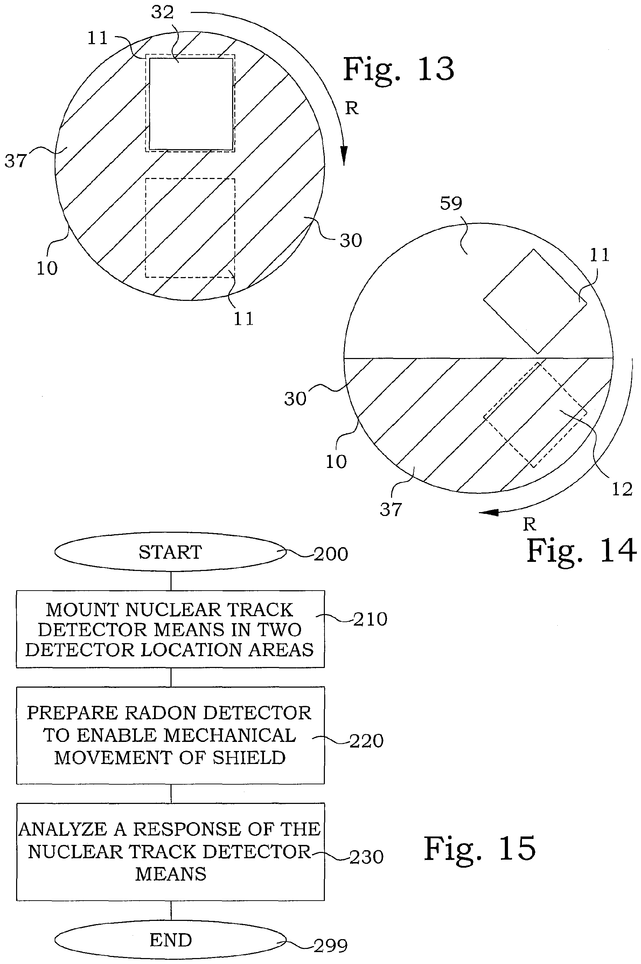

Fig. 13 illustrates another embodiment of a base portion 10 and a shield arrangement 30, rotationally movable. In this embodiment, the closing of one detector location area 11 is connected to the opening of the other detector location area 12. This is achieved by utilizing a common shield 37 with one hole 32, defining the area through which the detection of alpha particles can be performed.

Fig. 14 illustrates another embodiment of a base portion 10 and a shield arrangement 30, rotationally movable. In this embodiment, the closing of one detector location area 11 can be performed independently of the other detector location area 12. This is achieved by utilizing a common shield 37 occupying half the area of the base portion 10. An open side 59, defines the area through which the detection of alpha particles can be performed. The detector location areas 11 and 12 are situated in a non-rotationally symmetric manner, which enables the shield arrangement 30 to be rotated in such ways that none, either of the detector location areas 11 and 12 or both detector location areas 11 and 12 becomes coved by the shield 37.

Fig. 15 illustrates a flow diagram of steps of an embodiment of a method for measuring radon content. The procedure starts in step 200. In step 210, mounting of nuclear track detector means in at least two detector location areas in a radon detector is performed. The radon detector has a contained gas volume in diffusion contact with a surrounding. In step 220, the radon detector is prepared to enable, when the radon detector is placed at a measurement location, mechanically moving of a shield between a closed position and an open position, for each of the detector location areas. The shield in the closed position of a respective the detector location area prevents a line of sight between at least a part of the contained gas volume and the respective detector location area and the shield of the at least one shield in the open position of the particular detector position allows a line of sight between said at least a part of the contained gas volume and the respective detector location area.

In a particular embodiment, the step 220 of preparing further comprises preparing the radon detector to enable, when the radon detector is placed at a measurement location, registering, for at least one detector location area, a time for which the shield is in the open position. In a further particular embodiment, the registering is enabled to be performed for each of the detector location area separately. In another particular embodiment, the registering is enabled to comprise registering of a time for when the shield of the at least one shield is mechanically moved from the closed position to the open position and enabled to comprise registering of a time for when the shield of the at least one shield is mechanically moved from the open position to the closed position.

In a particular embodiment, the step of preparing further comprises preparing the radon detector to enable, when the radon detector is placed at a measurement location, storing the time in a memory intermittently, and retrieving the time from the memory when a detection period is ended.

In a particular embodiment, the step of preparing enables the mechanically moving to be performed to have the shield in the open position for at the most one detector location area simultaneously.

In step 230, a response of the nuclear track detector means is analyzed for presence of radon. The procedure ends in step 299.

The embodiments described above are merely given as examples, and it should be understood that the proposed technology is not limited thereto. It will be understood by those skilled in the art that various modifications, combinations and changes may be made to the embodiments without departing from the present scope as defined by the appended claims. In particular, different part solutions in the different embodiments can be combined in other configurations, where technically possible.

## Claims

1. A radon detector (1), comprising:
- a base portion (10);
- a cover portion (20), arranged for being removably attached to said base portion (10);
said cover portion (20), when being attached to said base portion (10), housing a contained gas volume (5A) between said cover portion (20) and said base portion (10);
said cover portion (20) and said base portion (10), when being attached to each other, allowing diffusion of gas between a surrounding (9) into said contained gas volume (5A);
said base portion (10) having at least two detector location areas (11, 12, 61,62), each of said two detector location areas being provided with a track detector means (90), enabling mechanical arranging of nuclear track detector means (90) to said base portion (10); and
- a shield arrangement (30), comprising at least one shield (37) and a shield actuator (38);
said shield actuator (38) being arranged for mechanically moving a shield (37) of said at least one shield between a closed position and an open position, for each of said detector location areas (11, 12, 61, 62);
wherein said shield (37) of said at least one shield in said closed position of a respective said detector location area (11, 12, 61, 62) prevents a line of sight between at least a part of said contained gas volume (5A) and said respective said detector location area (11, 12, 61, 62) and wherein said shield (37) of said at least one shield in said open position of said particular detector position allows a line of sight between said at least a part of said contained gas volume (5A) and said respective said detector location area (11, 12, 61, 62);
said shield actuator (38) being controllable from outside said contained gas volume (5A),
**characterized in that**
said shield actuator (38) being arranged for mechanically moving said shield (37) of said at least one shield between a closed position and an open position, for each of said detector location areas (11, 12, 61, 62), so that a closing of one detector location area is connected to an opening of another detector location area.

2. The radon detector according to claim 1, **characterized in that** said at least a part of said contained gas volume is a main part of said contained gas volume (5A).

3. The radon detector according to claim 1 or 2, **characterized by**
- a time stamp arrangement (40);
said time stamp arrangement (40) comprising a timer (41), a memory (42) and a memory reading port (44);
said shield arrangement (30) comprising a switch structure (46); wherein said switch structure (46) is arranged to interact with said timer (41) when said shield (37) of said at least one shield being mechanically moved from said closed position to said open position and when said shield (37) of said at least one shield being mechanically moved from said open position to said closed position, for at least one of said detector location areas (11, 12, 61, 62);
wherein said timer (41) is arranged to store information in said memory (42) representing at least an open time for said at least one of said detector location areas (11, 12, 61, 62);
said open time being a time difference between said shield (37) of said at least one shield being mechanically moved from said closed position to said open position and said shield (37) of said at least one shield being mechanically moved from said open position to said closed position.

4. The radon detector according to claim 3, **characterized in that** said time stamp arrangement (40) is arranged to store an open time for each said detector location area (11, 12, 61, 62) separately.

5. The radon detector according to claim 3 or 4, **characterized in that** said open time comprises a time for when said shield (37) of said at least one shield being mechanically moved from said closed position to said open position and a time for when said shield (37) of said at least one shield being mechanically moved from said open position to said closed position.

6. The radon detector according to any of the claims 3 to 5, **characterized in that** said switch structure (46) is an integrated part of said shield arrangement (30).

7. The radon detector according to any of the claims 3 to 6, **characterized in that** said switch structure (46) is arranged to mechanically interact with a part of the base portion (10) when said shield (37) of said at least one shield is in said open position.

8. The radon detector according to any of the claims 1 to 7, **characterized by** a spacer (19), defining a plane above a main inner surface of base portion (10), wherein said shield (37) of said at least one shield in a closed position is positioned juxtaposed to said plane, and preferably in contact with said spacer (19).

9. The radon detector according to any of the claims 1 to 8, **characterized in that** a remaining gas volume (5B) between said shield (37) of said at least one shield in said closed position and a respective detector location area (11, 12, 61, 62) in presence of a nuclear track detector means (90) is less than 10% of said contained gas volume (5A), preferably less than 3% of said contained gas volume (5A), and most preferably less than 1% of said contained gas volume (5A).

10. The radon detector according to any of the claims 1 to 9, **characterized in that** wherein said shield (37) of said at least one shield in said closed position of a respective said detector location area (11, 12, 61, 62) prevents alpha particles from radon decay within said at least a part of said contained gas volume (5A) to reach said respective said detector location area (11, 12, 61, 62) and wherein said shield (37) of said at least one shield in said open position of said particular detector position (11, 12, 61, 62) allows alpha particles from radon decay within said at least a part of said contained gas volume (5A) to reach said respective said detector location area (11, 12, 61, 62).

11. A method for measuring radon content, comprising:
- mounting (210) nuclear track detector means in at least two detector location areas in a radon detector having an contained gas volume in diffusion contact with a surrounding;
- preparing (220) said radon detector to enable, when said radon detector being placed at a measurement location, mechanically moving of a shield between a closed position and an open position, for each of said detector location areas;
wherein said shield in said closed position of a respective said detector location areas prevents a line of sight between at least a part of said contained gas volume and said respective said detector location area and wherein said shield of said at least one shield in said open position of said particular detector position allows a line of sight between said at least a part of said contained gas volume and said respective said detector location area; and
- analyzing (230) a response of said nuclear track detector means for presence of radon,
**characterized in that**
said mechanically moving enables that a closing of one detector location area is connected to an opening of another detector location area.

12. The method according to claim 11, **characterized in that** said step of preparing (220) further comprises preparing said radon detector to enable, when said radon detector being placed at a measurement location, registering, for at least one detector location area, a time for which said shield is in said open position.

13. The method according to claim 12, **characterized in that** said registering is enabled to be performed for each said detector location area separately.

14. The method according to claim 12 or 13, **characterized in that** registering is enabled to comprise registering of a time for when said shield of said at least one shield being mechanically moved from said closed position to said open position and enabled to comprise registering of a time for when said shield of said at least one shield being mechanically moved from said open position to said closed position.

15. The method according to any of the claims 11 to 14, **characterized in that** said step of preparing (220) further comprises preparing said radon detector to enable, when said radon detector being placed at a measurement location:
- storing said time in a memory intermittently; and
- retrieving said time from said memory when a detection period is ended.

## Patentansprüche

1. Radondetektor (1), umfassend:
- einen Basisabschnitt (10);
- einen Abdeckabschnitt (20), der angeordnet ist, um entfernbar an dem Basisabschnitt (10) angebracht zu werden;
wobei der Abdeckabschnitt (20), wenn er an dem Basisabschnitt (10) angebracht ist, ein enthaltenes Gasvolumen (5A) zwischen dem Abdeckabschnitt (20) und dem Basisabschnitt (10) aufnimmt;
wobei der Abdeckabschnitt (20) und der Basisabschnitt (10), wenn sie aneinander angebracht sind, die Diffusion von Gas zwischen einer Umgebung (9) in das enthaltene Gasvolumen (5A) ermöglichen;
wobei der Basisabschnitt (10) mindestens zwei Detektorlokalisierungszonen (11, 12, 61, 62) aufweist, wobei jede der zwei Detektorlokalisierungszonen mit einem Spurdetektormittel (90) versehen ist, das eine mechanische Anordnung des Kernspurdetektormittels (90) zu dem Basisabschnitt (10) ermöglicht; und
- eine Abschirmungsanordnung (30), die mindestens eine Abschirmung (37) und einen Abschirmungsaktor (38) umfasst;
wobei der Abschirmungsaktor (38) angeordnet ist, um eine Abschirmung (37) der mindestens einen Abschirmung für jeden der Detektorlokalisierungszonen (11, 12, 61, 62) mechanisch zwischen einer geschlossenen Position und einer offenen Position zu bewegen;
wobei die Abschirmung (37) der mindestens einen Abschirmung in der geschlossenen Position einer jeweiligen Detektorlokalisierungszone (11, 12, 61, 62) eine Sichtlinie zwischen mindestens einem Teil des enthaltenen Gasvolumens (5A) und der jeweiligen Detektorlokalisierungszone (11, 12, 61, 62) verhindert und wobei die Abschirmung (37) der mindestens einen Abschirmung in der offenen Position der bestimmten Detektorposition eine Sichtlinie zwischen dem mindestens einen Teil des enthaltenen Gasvolumens (5A) und der jeweiligen Detektorlokalisierungszone (11, 12, 61, 62) ermöglicht;
wobei der Abschirmungsaktor (38) von außerhalb des enthaltenen Gasvolumens (5A) steuerbar ist,
**dadurch gekennzeichnet, dass**
der Abschirmungsaktor (38) angeordnet ist, um die Abschirmung (37) der mindestens einen Abschirmung mechanisch zwischen einer geschlossenen Position und einer offenen Position für jede der Detektorlokalisierungszonen (11, 12, 61, 62) zu bewegen, sodass ein Verschließen einer Detektorlokalisierungszone mit einem Öffnen einer anderen Detektorlokalisierungszone verbunden ist.

2. Radondetektor nach Anspruch 1, **dadurch gekennzeichnet, dass** der mindestens eine Teil des enthaltenen Gasvolumens ein Hauptteil des enthaltenen Gasvolumens (5A) ist.

3. Radondetektor nach Anspruch 1 oder 2, **gekennzeichnet durch**
- eine Zeitstempelanordnung (40);
wobei die Zeitstempelanordnung (40) einen Zeitgeber (41), einen Speicher (42) und eine Speicherleseschnittstelle (44) umfasst;
wobei die Abschirmungsvorrichtung (30) eine Schaltstruktur (46) umfasst;
wobei die Schaltstruktur (46) angeordnet ist, um mit dem Zeitgeber (41) zu interagieren, wenn die Abschirmung (37) der mindestens einen Abschirmung für mindestens eine der Detektorlokalisierungszonen (11, 12, 61, 62) mechanisch aus der geschlossenen Position in die offene Position bewegt wird und wenn die Abschirmung (37) der mindestens einen Abschirmung mechanisch aus der offenen Position in die geschlossene Position bewegt wird;
wobei der Zeitgeber (41) angeordnet ist, um Informationen in dem Speicher (42) zu speichern, die mindestens eine offene Zeit für die mindestens eine der Detektorlokalisierungszonen (11, 12, 61, 62) darstellen;
wobei die offene Zeit ein Zeitunterschied zwischen der Abschirmung (37) der mindestens einen Abschirmung, die mechanisch aus der geschlossenen Position in die offene Position bewegt wird, und der Abschirmung (37) der mindestens einen Abschirmung ist, die mechanisch aus der offenen Position in die geschlossene Position bewegt wird.

4. Radondetektor nach Anspruch 3, **dadurch gekennzeichnet, dass** die Zeitstempelanordnung (40) angeordnet ist, um eine offene Zeit für jede Detektorlokalisierungszone (11, 12, 61, 62) separat zu speichern.

5. Radondetektor nach Anspruch 3 oder 4, **dadurch gekennzeichnet, dass** die offene Zeit eine Zeit dafür, wenn die Abschirmung (37) der mindestens einen Abschirmung mechanisch aus der geschlossenen Position in die offene Position bewegt wird, und eine Zeit dafür umfasst, wenn die Abschirmung (37) der mindestens einen Abschirmung mechanisch aus der offenen Position in die geschlossene Position bewegt wird.

6. Radondetektor nach einem der Ansprüche 3 bis 5, **dadurch gekennzeichnet, dass** die Schaltstruktur (46) ein integrierter Teil der Abschirmungsanordnung (30) ist.

7. Radondetektor nach einem der Ansprüche 3 bis 6, **dadurch gekennzeichnet, dass** die Schaltstruktur (46) angeordnet ist, um mit einem Teil des Basisabschnittes (10) mechanisch zu interagieren, wenn sich die Abschirmung (37) der mindestens einen Abschirmung in der offenen Position befindet.

8. Radondetektor nach einem der Ansprüche 1 bis 7, **gekennzeichnet durch** einen Abstandshalter (19), der eine Ebene oberhalb einer Hauptinnenfläche des Basisabschnittes (10) definiert, wobei die Abschirmung (37) der mindestens einen Abschirmung in einer geschlossenen Position neben dieser Ebene und vorzugsweise in Kontakt mit dem Abstandshalter (19) positioniert ist.

9. Radondetektor nach einem der Ansprüche 1 bis 8, **dadurch gekennzeichnet, dass** ein verbleibendes Gasvolumen (5B) zwischen der Abschirmung (37) der mindestens einen Abschirmung in der geschlossenen Position und einer jeweiligen Detektorlokalisierungszone (11, 12, 61, 62) bei Vorhandensein eines Kernspurdetektormittels (90) weniger als 10 % des enthaltenen Gasvolumens (5A) beträgt, vorzugsweise weniger als 3 % des enthaltenen Gasvolumens (5A) und am meisten bevorzugt weniger als 1 % des enthaltenen Gasvolumen (5A).

10. Radondetektor nach einem der Ansprüche 1 bis 9, **dadurch gekennzeichnet, dass** die Abschirmung (37) der mindestens einen Abschirmung in der geschlossenen Position einer jeweiligen Detektorlokalisierungszone (11, 12, 61, 62) verhindert, dass Alphapartikel von Radonzerfall innerhalb des mindestens einen Teils des enthaltenen Gasvolumens (5A) die jeweilige Detektorlokalisierungszone (11, 12, 61, 62) erreichen und wobei die Abschirmung (37) der mindestens einen Abschirmung in der offenen Position der bestimmten Detektorposition (11, 12, 61, 62) ermöglicht, dass Alphapartikel von Radonzerfall innerhalb des mindestens einen Teils des enthaltenen Gasvolumens (5A) die jeweilige Detektorlokalisierungszone (11, 12, 61, 62) erreichen.

11. Verfahren zum Messen von Radongehalt, umfassend:
- Montieren (210) von Kernspurdetektormitteln in mindestens zwei Detektorlokalisierungszonen in einem Radondetektor, der ein enthaltenes Gasvolumen in Diffusionskontakt mit einer Umgebung aufweist;
- Vorbereiten (220) des Radondetektors, um, wenn der Radondetektor an einer Messstelle platziert wird, das mechanische Bewegen einer Abschirmung zwischen einer geschlossenen Position und einer offenen Position für jede der Detektorlokalisierungszonen zu ermöglichen;
wobei die Abschirmung in der geschlossenen Position einer jeweiligen Detektorlokalisierungszone eine Sichtlinie zwischen mindestens einem Teil des enthaltenen Gasvolumens und der jeweiligen Detektorlokalisierungszone verhindert und wobei die Abschirmung der mindestens einen Abschirmung in der offenen Position der bestimmten Detektorposition eine Sichtlinie zwischen dem mindestens einen Teil des enthaltenen Gasvolumens und der jeweiligen Detektorlokalisierungszone ermöglicht; und
- Analysieren (230) einer Reaktion des Kernspurdetektormittels auf Vorhandensein von Radon,
**dadurch gekennzeichnet, dass**
das mechanische Bewegen ermöglicht, dass ein Schließen einer Detektorlokalisierungszone mit einem Öffnen einer anderen Detektorlokalisierungszone verbunden ist.

12. Verfahren nach Anspruch 11, **dadurch gekennzeichnet, dass** der Schritt des Vorbereitens (220) ferner das Vorbereiten des Radondetektors umfasst, um, wenn der Radondetektor an einer Messstelle platziert wird, das Registrieren einer Zeit, für die sich die Abschirmung in der offenen Position befindet, für mindestens eine Detektorlokalisierungszone zu ermöglichen.

13. Verfahren nach Anspruch 12, **dadurch gekennzeichnet, dass** das Registrieren so ermöglicht wird, dass es für jede Detektorlokalisierungszone separat durchgeführt wird.

14. Verfahren nach Anspruch 12 oder 13, **dadurch gekennzeichnet, dass** das Registrieren so ermöglicht wird, dass es das Registrieren einer Zeit umfasst, in der die Abschirmung der mindestens einen Abschirmung mechanisch aus der geschlossenen Position in die offene Position bewegt wird und ermöglicht wird, dass es das Registrieren einer Zeit umfasst, in der die Abschirmung der mindestens einen Abschirmung mechanisch aus der offenen Position in die geschlossene Position bewegt wird.

15. Verfahren nach einem der Ansprüche 11 bis 14, **dadurch gekennzeichnet, dass** der Schritt des Vorbereitens (220) ferner das Vorbereiten des Radondetektors umfasst, um Folgendes zu ermöglichen, wenn der Radondetektor an einer Messstelle platziert wird:
- intermittierendes Speichern der Zeit in einem Speicher; und
- Abrufen der Zeit aus dem Speicher, wenn eine Erfassungsperiode beendet ist.

## Revendications

1. Détecteur de radon (1), comprenant :
- une partie de base (10) ;
- une partie de couvercle (20), agencée pour être fixée de manière amovible à ladite partie de base (10) ;
ladite partie de couvercle (20), lorsqu'elle est fixée à ladite partie de base (10), logeant un volume de gaz contenu (5A) entre ladite partie de couvercle (20) et ladite partie de base (10) ;
ladite partie de couvercle (20) et ladite partie de base (10), lorsqu'elles sont fixées l'une à l'autre, permettant la diffusion de gaz entre un environnement (9) dans ledit volume de gaz contenu (5A) ;
ladite partie de base (10) ayant au moins deux zones d'emplacement de détecteur (11, 12, 61, 62), chacune desdites deux zones d'emplacement de détecteur étant pourvue d'un moyen de détection de traces (90), permettant un agencement mécanique de moyen de détection de traces nucléaires (90) à ladite partie de base (10) ; et
- un agencement de protection (30), comprenant au moins un élément de protection (37) et un actionneur de protection (38) ;
ledit actionneur de protection (38) étant agencé pour déplacer mécaniquement un élément de protection (37) dudit au moins un élément de protection entre une position fermée et une position ouverte, pour chacune desdites zones d'emplacement de détecteur (11, 12, 61, 62) ;
dans lequel ledit élément de protection (37) dudit au moins un élément de protection dans ladite position fermée d'une dite zone d'emplacement de détecteur respective (11, 12, 61, 62) empêche une ligne de visée entre au moins une partie dudit volume de gaz contenu (5A) et ladite dite zone d'emplacement de détecteur respective (11, 12, 61, 62) et dans lequel ledit élément de protection (37) dudit au moins un élément de protection dans ladite position ouverte de ladite position de détecteur particulière permet une ligne de visée entre ladite au moins une partie dudit volume de gaz contenu (5A) et ladite zone d'emplacement de détecteur respective (11, 12, 61, 62) ;
ledit actionneur de protection (38) pouvant être commandé de l'extérieur dudit volume de gaz contenu (5A),
**caractérisé en ce que**
ledit actionneur de protection (38) est agencé pour déplacer mécaniquement ledit élément de protection (37) dudit au moins un élément de protection entre une position fermée et une position ouverte, pour chacune desdites zones d'emplacement de détecteur (11, 12, 61, 62), de sorte qu'une fermeture d'une zone d'emplacement de détecteur est reliée à une ouverture d'une autre zone d'emplacement de détecteur.

2. Détecteur de radon selon la revendication 1, **caractérisé en ce que** ladite au moins une partie dudit volume de gaz contenu est une partie principale dudit volume de gaz contenu (5A).

3. Détecteur de radon selon la revendication 1 ou 2, **caractérisé par**
- un dispositif d'horodatage (40) ;
ledit dispositif d'horodatage (40) comprenant un temporisateur (41), une mémoire (42) et un port de lecture de mémoire (44) ;
ledit agencement de protection (30) comprenant une structure de commutation (46) ;
dans lequel ladite structure de commutation (46) est agencée pour interagir avec ledit temporisateur (41) lorsque ledit élément de protection (37) dudit au moins un élément de protection est déplacé mécaniquement de ladite position fermée à ladite position ouverte et lorsque ledit élément de protection (37) dudit au moins un élément de protection est déplacé mécaniquement de ladite position ouverte à ladite position fermée, pour au moins une desdites zones d'emplacement de détecteur (11, 12, 61, 62) ;
dans lequel ledit temporisateur (41) est agencé pour stocker des informations dans ladite mémoire (42) représentant au moins un temps d'ouverture pour ladite au moins une desdites zones d'emplacement de détecteur (11, 12, 61, 62) ;
ledit temps d'ouverture étant une différence de temps entre ledit élément de protection (37) dudit au moins un élément de protection étant déplacé mécaniquement de ladite position fermée à ladite position ouverte et ledit élément de protection (37) dudit au moins un élément de protection étant déplacé mécaniquement de ladite position ouverte à ladite position fermée.

4. Détecteur de radon selon la revendication 3, **caractérisé en ce que** ledit dispositif d'horodatage (40) est agencé pour stocker séparément un temps d'ouverture pour chaque dite zone d'emplacement de détecteur (11, 12, 61, 62).

5. Détecteur de radon selon la revendication 3 ou 4, **caractérisé en ce que** ledit temps d'ouverture comprend un instant pendant lequel ledit élément de protection (37) dudit au moins un élément de protection est déplacé mécaniquement de ladite position fermée à ladite position ouverte et un instant pendant lequel ledit élément de protection (37) dudit au moins un élément de protection est déplacé mécaniquement de ladite position ouverte à ladite position fermée.

6. Détecteur de radon selon l'une quelconque des revendications 3 à 5, **caractérisé en ce que** ladite structure de commutation (46) fait partie intégrante dudit agencement de protection (30).

7. Détecteur de radon selon l'une quelconque des revendications 3 à 6, **caractérisé en ce que** ladite structure de commutation (46) est agencée pour interagir mécaniquement avec une partie de la partie de base (10) lorsque ledit élément de protection (37) dudit au moins un élément de protection est dans ladite position ouverte.

8. Détecteur de radon selon l'une quelconque des revendications 1 à 7, **caractérisé par** une entretoise (19), définissant un plan au-dessus d'une surface intérieure principale de la partie de base (10), dans lequel ledit élément de protection (37) dudit au moins un élément de protection en position fermée est positionné de manière juxtaposée audit plan, et de préférence en contact avec ladite entretoise (19).

9. Détecteur de radon selon l'une quelconque des revendications 1 à 8, **caractérisé en ce qu'**un volume de gaz restant (5B) entre ledit élément de protection (37) dudit au moins un élément de protection dans ladite position fermée et une zone d'emplacement de détecteur respective (11, 12, 61, 62) en présence d'un moyen de détection de traces nucléaires (90) est inférieur à 10 % dudit volume de gaz contenu (5A), de préférence inférieur à 3 % dudit volume de gaz contenu (5A), et de manière davantage préférée inférieur à 1 % dudit volume de gaz contenu (5A).

10. Détecteur de radon selon l'une quelconque des revendications 1 à 9, **caractérisé en ce que** ledit élément de protection (37) dudit au moins un élément de protection dans ladite position fermée d'une dite zone d'emplacement de détecteur respective (11, 12, 61, 62) empêche les particules alpha provenant de la désintégration du radon à l'intérieur de ladite au moins une partie dudit volume de gaz contenu (5A) d'atteindre ladite zone d'emplacement de détecteur respective (11, 12, 61, 62) et dans lequel ledit élément de protection (37) dudit au moins un élément de protection dans ladite position ouverte de ladite position de détecteur particulière (11, 12, 61, 62) permet aux particules alpha provenant de la désintégration du radon à l'intérieur de ladite au moins une partie dudit volume de gaz contenu (5A) d'atteindre ladite zone d'emplacement de détecteur respective (11, 12, 61, 62).

11. Procédé de mesure de la teneur en radon, comprenant :
- le montage (210) d'un moyen de détection de traces nucléaires dans au moins deux zones d'emplacement de détecteur dans un détecteur de radon ayant un volume de gaz contenu en contact de diffusion avec un environnement ;
- la préparation (220) dudit détecteur de radon pour permettre, lorsque ledit détecteur de radon est placé à un emplacement de mesure, de déplacer mécaniquement un élément de protection entre une position fermée et une position ouverte, pour chacune desdites zones d'emplacement de détecteur ;
dans lequel ledit élément de protection dans ladite position fermée desdites zones d'emplacement de détecteur respectives empêche une ligne de visée entre au moins une partie dudit volume de gaz contenu et ladite dite zone d'emplacement de détecteur respective et dans lequel ledit élément de protection dudit au moins un élément de protection dans ladite position ouverte de ladite position de détecteur particulière permet une ligne de visée entre ladite au moins une partie dudit volume de gaz contenu et ladite dite zone d'emplacement de détecteur respective ; et
- l'analyse (230) d'une réponse dudit moyen de détection de traces nucléaires pour la présence de radon,
**caractérisé en ce que**
ledit déplacement mécanique permet qu'une fermeture d'une zone d'emplacement de détecteur soit reliée à une ouverture d'une autre zone d'emplacement de détecteur.

12. Procédé selon la revendication 11, **caractérisé en ce que** ladite étape de préparation (220) comprend en outre la préparation dudit détecteur de radon pour permettre, lorsque ledit détecteur de radon est placé à un emplacement de mesure, d'enregistrer, pour au moins une zone d'emplacement de détecteur, un instant pendant lequel ledit élément de protection est dans ladite position ouverte.

13. Procédé selon la revendication 12, **caractérisé en ce que** ledit enregistrement peut être effectué séparément pour chaque dite zone d'emplacement de détecteur.

14. Procédé selon la revendication 12 ou 13, **caractérisé en ce que** l'enregistrement peut comprendre l'enregistrement d'un instant pendant lequel ledit élément de protection dudit au moins un élément de protection est déplacé mécaniquement de ladite position fermée à ladite position ouverte et peut comprendre l'enregistrement d'un instant pendant lequel ledit élément de protection dudit au moins un élément de protection est déplacé mécaniquement de ladite position ouverte à ladite position fermée.

15. Procédé selon l'une quelconque des revendications 11 à 14, **caractérisé en ce que** ladite étape de préparation (220) comprend en outre la préparation dudit détecteur de radon pour permettre, lorsque ledit détecteur de radon est placé à un emplacement de mesure :
- le stockage dudit instant dans une mémoire par intermittence ; et
- la récupération dudit instant à partir de ladite mémoire lorsqu'une période de détection est terminée.
